# EUROPEAN PATENT APPLICATION

(11) **EP 2 214 017 A1**
(43) Date of publication of application: **04.08.2010**
(21) Application number: 08842461.9
(22) Date of filing: 22.10.2008
(51) Int. Cl.: G01N 33/53, G01N 33/531, G01N 33/543, G01N 33/545, G01N 33/553, G01N 33/577

(54) **METHOD AND KIT FOR MEASUREMENT OF ACROLEIN ADDUCT IN SAMPLE UTILIZING AGGLUTINATION REACTION OF IMMUNOLOGICAL MICROPARTICLE**

(30) Priority: 22.10.2007 JP 2007274325
(71) Applicant: Alfresa Pharma Corporation, Osaka-shi, Osaka 540-8575 (JP); NOF Corporation, 20-3 Ebisu 4-chome, Shibuya-ku Tokyo 150-6019 (JP)
(72) Inventor: YANAGIYA, Mari, Ibaraki-shi Osaka 567-0806 (JP); TANAKA, Mutsumi, Ibaraki-shi Osaka 567-0806 (JP); KOSAKA, Mieko, Ibaraki-shi Osaka 567-0806 (JP); ENOMOTO, Masayasu, Ibaraki-shi Osaka 567-0806 (JP); YAMADA, Satoshi, Tsukuba-shi Ibaraki 300-2635 (JP); NAKASHIMA, Fumio, Tsukuba-shi Ibaraki 300-2635 (JP)
(74) Representative: MacLean, Martin Robert
(86) International application number: PCT/JP2008/069587
(87) International publication number: WO 2009/054538

(57) **Abstract**

The method for measuring an acrolein adduct present in a sample according to the present invention comprising the steps of: (a) mixing a sample containing the acrolein adduct with a solution comprising an immunoglobulin that specifically recognizes the acrolein adduct; (b) adding to the mixture obtained in the step (a), a solution comprising microparticles to which a substance that specifically binds to the immunoglobulin that specifically recognizes the acrolein adduct and a blocking agent have been bound, and mixing them; and (c) measuring an extent of an agglutination reaction of the microparticles in the mixture obtained in the step (b), wherein the extent of the agglutination reaction being decreased relative to an amount of the acrolein adduct in the sample. The present invention enables a measurement of an acrolein adduct without requiring a complex operation.

## Description

### Technical Field

The present invention relates to an immunoassay using microparticles to which a substance has been bound. In particular, the present invention relates to an immunoassay of an acrolein adduct using an antigen-antibody reaction for use mainly in the industrial, environmental, and clinical laboratory test fields, and to a reagent kit for immunoassay.

### Background Art

In recent years, automation in various types of tests such as clinical laboratory tests and reduction in the assay time thereof have been tried. As a method of these tests, an assay utilizing an immune reaction is widely used for measurement of a substance in a biological sample. Examples of the immunoassay include many methods such as RIA, EIA, immunonephelometry, latex agglutination, colloidal gold agglutination, and immunochromatography. Among such methods, the latex agglutination and the colloidal gold agglutination are capable of measurement in a homogeneous system in which the separation or washing operation of a reaction mixture is not required, and therefore suitable for automation of determination and short-time assay. In particular, since colloidal gold particles have a size of 5 nm to 100 nm, which is smaller than the size of latex particles, colloidal gold particles can be used in an assay of a tracer substance (Japanese Laid-Open Patent Publication Nos. 2005-283250 and 2004-325192).

The main reactive components in these measurement methods are microparticles such as latex particles and colloidal gold particles to which substances that specifically reacts (for example, bind) with analytes are bound. The binding of a specific binding substance such as an antibody that is present on the microparticles with an analyte cause the microparticles to agglutinate. This agglutination occurs in a manner depending on the amount of the analyte, and thus a mechanical measurement of this phenomenon enables the mass of the analyte to be calculated.

Examples of known usable carrier particles for such a measurement using agglutination include, in addition to the aforementioned latex particles and colloidal gold particles, polyethylene glycol-bound microparticle carriers (Japanese Laid-Open Patent Publication No. 2004-300253), microcarriers composed of a styrene derivative and a vinyl compound (Japanese Laid-Open Patent Publication No. 7-133232), polymeric particles with a surface having anisotropy (Japanese Laid-Open Patent Publication No. 10-87841), and a liposome prepared by a specific means (Japanese Laid-Open Patent Publication Nos. 9-5325 and 9-229937).

Various researches have been made with respect to reagents used in combination with carrier particles to enhance measurement accuracy. For example, a measurement method that uses latex particles on which an antibody against a high-concentration C-reactive protein is supported in combination with a compound that has a phosphorylcholine group and a cationic group is known (Japanese Laid-Open Patent Publication No. 2001-318099). In addition, there is an agglutination immunoassay that uses a polymer produced by homopolymerizing a monomer that has a phosphorylcholine group and a vinyl group or a polymer produced by copolymerizing a monomer that has a phosphorylcholine group and a vinyl group with a monomer that has a vinyl group (WO02/018953). Furthermore, there is an assay reagent, as a reagent for diagnosing syphilis infection, that uses an antigen-supporting carrier in combination with a copolymer that has a segment derived from 2-methacryloyloxyethyl phosphorylcholine and a segment derived from a hydrophilic monomer (Japanese Laid-Open Patent Publication Nos. 2007-155623 and 2007-155624).

However, medicaments, chemical substances, and like analytes having low molecular weights in particular have a small number of sites available where specific binding substances and analytes can bind, and an agglutination reaction is thus unlikely to occur, making it difficult to construct a homogeneous measurement system using an agglutination reaction. Therefore, techniques, e.g., introducing an analyte or a competitor to which a plurality of haptens are bound into a reaction system, need to be employed.

### Disclosure of Invention

It is an object of the present invention to provide a method for homogeneously measuring an acrolein adduct that is a low molecular weight substance with which it is difficult to construct a measurement system using an aforementioned agglutination reaction and to provide a measurement kit therefore.

In the present invention, more convenient measurement of the low molecular weight acrolein adduct can be achieved without adding a specific competitor by mixing a sample containing an analyte acrolein adduct; a solution containing an immunoglobulin that specifically recognizes the acrolein adduct; and a solution containing microparticles to which a substance that specifically binds to the immunoglobulin that specifically recognizes the acrolein adduct and a blocking agent have been bound.

The present invention provides a method for measuring an acrolein adduct present in a sample, the method comprising the steps of:
(a) mixing a sample containing the acrolein adduct with a solution comprising an immunoglobulin that specifically recognizes the acrolein adduct;
(b) adding to the mixture obtained in the step (a), a solution comprising microparticles to which a substance that specifically binds to the immunoglobulin that specifically recognizes the acrolein adduct and a blocking agent have been bound, and mixing them; and
(c) measuring an extent of an agglutination reaction of the microparticles in the mixture obtained in the step (b), wherein the extent of the agglutination reaction being decreased relative to an amount of the acrolein adduct in the sample.

In one embodiment, the immunoglobulin that specifically recognizes the accrolein adduct is a monoclonal antibody that specifically recognizes a formyldehydropiperidine structure of the acrolein adduct.

In a certain embodyment, the substance that specifically binds to the immunoglobulin is an antibody that specifically binds to the immunoglobulin, and is preferably a monoclonal antibody.

In a further embodiment, the blocking agent is a common blocking agent that is used in the preparation of immunoreagents, and is preferably bovine serum albumin.

In a further embodiment, the solution containing microparticles to which a substance that specifically binds to the immunoglobulin and a blocking agent have been bound contains a buffer and a protein, and the protein is preferably bovine serum albumin.

In one embodiment, the microparticles are latex or gold colloid.

The present invention provides a reagent kit for measurement of an acrolein adduct, comprising,
a first reagent comprising an immunoglobulin that specifically recognizes the acrolein adduct, and
a second reagent comprising microparticles to which a substance that specifically binds to the immunoglobulin that specifically recognizes the acrolein adduct and a blocking agent have been bound.

In a further embodiment, the second reagent contains a buffer and a protein, and the protein is preferably bovine serum albumin.

According to the present invention, more convenient measurement of the acrolein adduct in a homogeneous system can be achieved by using an immunoglobulin that specifically recognizes an analyte acrolein adduct and microparticles to which a substance that specifically binds to the immunoglobulin and a blocking agent have been bound. The method of the present invention uses a convenient, homogeneous measurement system and is thus suitably applicable to a measurement using an autoanalyzer. Accordingly, the present invention shortens the time for an acrolein adduct measurement and reduces labor.

### Brief description of the Drawings

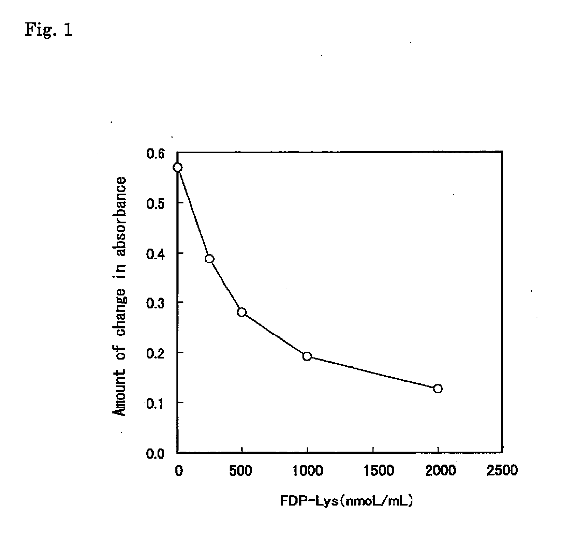

FIG. 1 is a graph showing the relationship between the concentration of Nε-(3-formyl-3,4-dehydropiperidino)-lysine (FDP-Lys) and the amount of change in absorbance (calibration curve for measuring acrolein adduct).

### Description of the Preferred Embodiments

Acrolein (CH₂= CH-CHO), which is the analyte in the present invention, is a chemical substance generated not only from the combustion of petroleum, coal, wood, and plastics, but also from cigarette smoke, exhaust gas, and heating of oils and fats, and is known to be highly cytotoxic. It was found recently that acrolein is also generated from the peroxidation of lipids, and its in vivo effect has been a concern. Acrolein is an analyte targeted in the clinical laboratory test field, e.g., as a marker of oxidative stress, in particular, a marker of cerebral apoplexy and asymptomatic cerebral infarction, in the clinical laboratory test field (see Japanese Laid-Open Patent Publication No. 2005-334476).

Acrolein is usually bound to the ε-amino group of lysine in a protein and present as a substance having a formyldehydropiperidino-lysine structure (sometimes referred to as FDP-Lys). In the present invention, a substance that has this structure is referred to as an acrolein adduct (sometimes referred to as ACR-Lys), and such an acrolein adduct is regarded as an analyte.

In the present invention, examples of samples containing an analyte acrolein adduct used in a measurement include biological samples such as blood, plasma, serum, urine, feces (in suspension), cerebrospinal fluid, and ascites fluid; and those collected from the environment or extracts thereof.

Immunoglobulins that specifically recognize analyte acrolein adducts (specifically bind to acrolein adducts) are not particularly limited, and are preferably polyclonal antibodies or monoclonal antibodies against acrolein adducts because they bind highly specifically to acrolein adducts. More preferable are monoclonal antibodies that specifically recognize the formyldehydropiperidine structure of acrolein adducts. Examples of polyclonal antibodies include antiserums against ACR-Lys as described in Japanese Laid-Open Patent Publication No. 11-80023. Usable examples of monoclonal antibodies include monoclonal antibodies as described in Japanese Laid-Open Patent Publication No. 11-147899 can be used.

Examples of substances that specifically bind to the aforementioned immunoglobulins include protein A and protein G that can specifically bind to immunoglobulins, and antibodies that recognize immunoglobulins. Antibodies that recognize immunoglobulins are preferable because they bind highly specifically. Furthermore, monoclonal antibodies that recognize immunoglobulins are preferable.

Blocking agents for use in the present invention may be common blocking agents that are usually used in the preparation of immunoreagents. For example, bovine serum albumin is preferable.

In the present invention, the microparticles to which a substance that specifically binds to the immunoglobulin that specifically recognizes an acrolein adduct, and a blocking agent are to be bound can be any microparticles that can be usually used for an immunoassay reagents. For example, latex and metal colloid are preferably used. In the case of metal colloid, gold colloid is preferable in view of generally ease to use. Commercially available colloidal gold particles may be used, or colloidal gold particles may be prepared by a method commonly used by those skilled in the art (e.g., a method of reducing chloroauric acid with sodium citrate). The particle size of the colloidal gold particles is usually in the range of 10 nm to 100 nm, preferably in the range of 30 nm to 60 nm.

Microparticles to which a substance that specifically binds to the aforementioned immunoglobulin and a blocking agent have been bound (hereinafter sometimes referred to as bound microparticles) can be prepared for use in the method of the present invention, for example, in the following manner if colloidal gold particles are used as the microparticles. First, usually 0.1 mg to 100 mg, preferably 1 mg to 10 mg, of a substance (e.g., an antibody) that specifically binds to an immunoglobulin that specifically recognizes an acrolein adduct is added to 1 L of a colloidal solution containing gold particles (having an absorbance at 540 nm of about 2.0), and the mixture is stirred under refrigeration or at room temperature for 5 minutes to 24 hours. Then, the mixture is subjected to blocking with bovine serum albumin or the like and centrifuged, and thus the desired bound microparticles (bound colloidal gold particles in this case) can be obtained. The obtained microparticles are dispersed in a buffer solution to attain a concentration required for measurement. The pH of the buffer solution is preferably 5 to 9, and the concentration thereof is preferably 1 to 100 mM. For example, a phosphate buffer solution, a Tris-HCl buffer solution, a succinate buffer solution, or a Good's buffer solution such as glycylglycine, MES (2-(N-morpholino)ethanesulfonic acid), HEPES (2-[4-(2-hydroxyethyl)-1-piperazinyl]ethanesulfonic acid), TES (N-tris (hydroxymethyl)methyl-2-aminoethanesulfonic acid), MOPS (3-(N-morpholino)propanesulfonic acid), PIPES (piperazine-1,4-bis(2-ethanesulfonic acid)), or Bis-Tris (bis(2-hydroxyethyl)iminotris (hydroxymethyl)methane) is preferably used as the buffer solution.

The buffer solution may contain additives, for example, sugars and sugar alcohols, sodium azide, albumin, salts such as sodium chloride, and antiseptics, as necessary Examples of the sugars and sugar alcohols include glucose, mannose, saccharose, lactose, maltose, mannitol, and sorbitol. The concentration thereof is preferably 0.01 to 10 w/v%. As for the albumin, bovine serum albumin (BSA) is preferably used, and the concentration thereof is preferably 0.001 to 1 w/v%. As for the antiseptics, sodium azide is preferably used, and the concentration thereof is preferably 0.01 to 0.5 w/v%. Examples of other additives include Tween 20, polyethylene glycol lauryl ether, 5-bromosalicylic acid, sodium salicylate, sodium benzoate, sodium benzenesulfonate, phenol, and thymol.

For the binding reaction between the analyte acrolein adduct and an immunoglobulin that specifically recognizes the acrolein adduct and for the agglutination reaction between the resulting reaction solution and microparticles to which a substance that specifically binds to the immunoglobulin and a blocking agent have been bound, reaction conditions such as the reaction temperature, the pH, the type of the buffer solution, the type of the coexistent salt and the concentration thereof, and the other coexistent substances are the same as those used for conventional immunological reactions. For example, in order to promote the reactions, a water-soluble polymer such as polyethylene glycol, polyvinyl alcohol, dextran, or sodium chondroitin sulfate may be added to the reaction system, as is commonly performed.

In the present invention, the method for measuring an analyte acrolein adduct present in a sample includes the steps of:
(a) mixing a sample containing the acrolein adduct with a solution comprising an immunoglobulin that specifically recognizes the acrolein adduct;
(b) adding to the mixture obtained in the step (a), a solution comprising microparticles to which a substance that specifically binds to the immunoglobulin that specifically recognizes the acrolein adduct and a blocking agent have been bound (i.e., bound microparticles), and mixing them; and
(c) measuring an extent of an agglutination reaction of the microparticles in the mixture obtained in the step (b), wherein the extent of the agglutination reaction being decreased relative to an amount of the acrolein adduct in the sample.

In the method, the agglutination reaction of the bound microparticles of latex or gold colloid occurs in a manner depending on the concentration of the analyte acrolein adduct, and the extent of the reaction is mechanically measured. In the method of the present invention, the extent of the agglutination reaction is decreased relative to the amount of the acrolein adduct in the sample.

For example, the method of the present invention is performed in the following manner: a sample containing an analyte acrolein adduct or a diluted liquid obtained by appropriately diluting this sample with a buffer solution or the like is mixed with a solution containing an immunoglobulin that specifically recognizes the acrolein adduct to form a complex; then, a solution containing microparticles to which a substance that specifically binds to the immunoglobulin that specifically recognizes the acrolein adduct and a blocking agent have been bound (bound microparticles) obtained in the above-described manner is added to the reaction solution containing the complex and they are mixed to cause an agglutination reaction in a manner depending on the concentration of the analyte. When gold colloid is used as the microparticles, a change in absorbance at a predetermined wavelength due to this agglutination reaction is determined. The amount of the analyte in the sample can be easily found by applying the results of the determination to a calibration curve created beforehand. The calibration curve represents the relationship between the change in the absorbance due to the colloidal gold agglutination reaction and the amount of the analyte. It should be noted that a qualitative analysis and a semi-quantitative analysis can also be performed by determining whether the absorbance change is above or below a specific value.

When gold colloid is used as the microparticle, both a single wavelength measurement and a dual wavelength measurement may be used to determine the change in the absorbance after the start of the reaction. When the dual wavelength measurement is used, the change in the absorbance is determined at the first wavelength of 610 nm to 800 nm, preferably 630 nm to 750 nm, and the second wavelength of 360 nm to 580 nm, preferably 500 nm to 550 nm. When the single wavelength measurement is used, the change in the absorbance can be determined at a wavelength in the wavelength region of either one of the first wavelength or the second wavelength used in the above-described dual wavelength measurement. In the method of the present invention, the change in the absorbance refers to values obtained by the two measurement methods described below, and either of the values can be used:
(1) the absorbance of the reaction solution is measured twice at an appropriate interval after the start of the reaction, and the difference between the two measured values is used as the change in the absorbance; or
(2) the absorbance of the reaction solution is continuously measured after the start of the reaction, and the rate of change in the absorbance per unit time (in some cases, the maximum rate of change) is used as the change in the absorbance.

A spectrophotometer, a microplate reader, a biochemical automatic analyzer, and the like can be used in the above-described measurement. In particular, a number of samples can be determined in a short period of time by applying the method of the present invention to the measurement with the biochemical automatic analyzer.

According to the present invention, a reagent kit for measuring an acrolein adduct for use with the method of the present invention is provided. The kit includes: a first reagent comprising an immunoglobulin that specifically recognizes the acrolein adduct; and a second reagent comprising microparticles to which a substance that specifically binds to the immunoglobulin that specifically recognizes the acrolein adduct and a blocking agent have been bound.

It is preferable that the second reagent comprising microparticles to which a substance that specifically binds to the immunoglobulin and a blocking agent have been bound further comprises a buffer and a protein. Bovine serum albumin is preferably used as the protein.

The above described reagents may be provided in any form, and preferably are provided in the form where the reagents are individually sealed and packaged. The above-described kit may include a reference standard of the analyte for use in creation of a calibration curve, a buffer solution in which each substance is dissolved on use to prepare a solution having an appropriate concentration, instructions for use of the kit, and the like.

### Examples

Hereinafter, the present invention will be described even more specifically by way of examples. However, the present invention is not limited by the examples.

### (Example 1: Preparation of colloidal gold solution)

First, 2 mL of a 10 w/v% chloroauric acid solution was added to 1 L of distilled water at 95°C under stirring, and after one minute, 10 mL of a 2 w/v% sodium citrate solution was added thereto. The resulting mixture was stirred for further 20 minutes and then cooled to 30°C. After cooling, the pH was adjusted to 7.1 with 0.1 w/v% potassium carbonate.

### (Example 2: Preparation of rat anti-mouse IgG monoclonal antibody and bovine serum albumin-bound colloidal gold reagent)

A rat anti-mouse IgG monoclonal antibody (Production of Antibodies, Reagents for Immunology and Services) was diluted with 10 mM HEPES (pH 7.1) containing 0.05 w/v% of sodium azide to a concentration of 20 µg/mL. Then, 50 mL of this solution was added to about 1 L of the colloidal gold solution prepared in Example 1, and the mixture was stirred under refrigeration for 2 hours. To this mixture, 110 mL of 10 mM HEPES (pH 7.1) containing 0.5 w/v% of bovine serum albumin, 5.46 w/v% of mannitol, and 0.05 w/v% of sodium azide was added, and the resulting mixture was stirred at 37°C for 90 minutes, and thereby a blocking treatment was performed. The mixture was centrifuged at 8000 rpm for 40 minutes to remove the supernatant. Then, about 1 L of 5 mM HEPES (pH 7.5) containing 3 w/v% of mannitol, 0.1 w/v% of BSA, and 0.05 w/v% of sodium azide (solution A) was added to the mixture, and the antibody-bound gold colloid was dispersed. Thereafter, centrifugation was performed at 8000 rpm for 40 minutes to remove the supernatant. Then, the solution A was added to disperse the antibody-bound gold colloid so that the total amount of the resulting solution was 70 mL. Thus, a rat anti-mouse IgG monoclonal antibody-bound colloidal gold solution was prepared.

Then, 280 mL of solution A was added to 70 mL of the rat anti-mouse IgG monoclonal antibody and bovine serum albumin-bound colloidal gold solution to prepare a rat anti-mouse IgG monoclonal antibody-bound colloidal gold reagent.

### (Example 3: Preparation of first reagent for measuring acrolein adduct)

A first reagent for measuring an acrolein adduct was prepared by adding 1.25 µg/mL of an anti-acrolein monoclonal antibody (NOF Corporation) and about 1.0 to 2.5 w/v% of polyethylene glycol serving as a reaction accelerator to a solution of 0.2 M PIPES (pH 6.5) containing 1.0 w/v% sodium chloride, 0.5 w/v% EDTA, and 0.35 w/v% polyoxyethylene lauryl ether.

### (Example 4: Measurement of acrolein adduct)

In this example, the first reagent for measuring an acrolein adduct prepared in Example 3 was used as a first reagent, and the rat anti-mouse IgG monoclonal antibody and bovine serum albumin-bound gold colloidal reagent prepared in Example 2 was used as a second reagent. As the acrolein adduct, a lysine compound in which acrolein is bound to the ε-amino group of lysine, Nε-(3-formyl-3,4-dehydropiperidino)-lysine (NOF Corporation) (referred to as FDP-Lys) is used. Samples were prepared by dissolving the adduct in a 1.0% NaCl solution at the concentrations of 0, 250, 500, 1000, 2000, and 4000 nmol/mL. Then, 170 µL of the first reagent was added to 6 µL of an FDP-Lys-containing sample, and the mixture warmed at 37°C for about 5 minutes. After warming, 85 µL of the second reagent was added and allowed to react at 37°C, and the amount of change in absorbance was measured by a Hitachi 7070 automatic analyzer at photometric points from 18 to 31 at wavelengths of 546 nm and 660 nm. FIG. 1 shows the relationship between the FDP-Lys concentration and the amount of change in absorbance.

As shown in FIG. 1, the amount of change in absorbance due to the agglutination reaction was changed (decreased) in a manner depending on the concentration of analyte FDP-Lys. In other words, it can be found that the amount of an acrolein adduct, which is the analyte contained in a sample, can be quantified by measuring the amount of change in absorbance due to the agglutination reaction and performing a comparison with the calibration curve.

### Industrial Applicability

The method of the present invention is a method for measuring an acrolein adduct performed in a homogenous system that does not require B/F separation, and is therefore also very suitable for automation. For example, automatic analyzers that are in widespread use in the clinical laboratory test field are usable. Therefore, the method of the present invention is suitable as an immunoassay for an acrolein adduct using an antigen-antibody reaction for use in the industrial, environmental, and clinical laboratory test fields.

## Claims

1. A method for measuring an acrolein adduct present in a sample, the method comprising the steps of:
(a) mixing a sample containing the acrolein adduct with a solution comprising an immunoglobulin that specifically recognizes the acrolein adduct;
(b) adding, to the mixture obtained in the step (a), a solution comprising microparticles to which a substance that specifically binds to the immunoglobulin that specifically recognizes the acrolein adduct and a blocking agent have been bound, and mixing them; and
(c) measuring an extent of an agglutination reaction of the microparticles in the mixture obtained in the step (b), wherein the extent of the agglutination reaction being decreased relative to an amount of the acrolein adduct in the sample.

2. The method of claim 1, wherein the immunoglobulin is a monoclonal antibody that specifically recognizes a formyldehydropiperidine structure of the acrolein adduct.

3. The method of claim 1 or 2, wherein the substance that specifically binds to the immunoglobulin is an antibody against the immunoglobulin.

4. The method of claim 3, wherein the antibody is a monoclonal antibody.

5. The method of any one of claims 1 to 4, wherein the blocking agent is bovine serum albumin.

6. The method of any one of claims 1 to 5, wherein the solution comprising microparticles further comprises a buffer and a protein.

7. The method of claim 6, wherein the protein is bovine serum albumin.

8. The method of any one of claims 1 to 7, wherein the microparticles are latex or gold colloid.

9. A reagent kit for measuring an acrolein adduct, comprising:
a first reagent comprising an immunoglobulin that specifically recognizes the acrolein adduct; and
a second reagent comprising microparticles to which a substance that specifically binds to the immunoglobulin that specifically recognizes the acrolein adduct and a blocking agent have been bound.

10. The kit of claim 9, the second reagent comprising microparticles to which a substance that specifically binds to the immunoglobulin and a blocking agent have been bound further comprises a buffer and a protein.

11. The kit of claim 10, wherein the protein is bovine serum albumin.
